# EUROPÄISCHE PATENTSCHRIFT

(11) **EP 2 874 912 B1**
(45) Veröffentlichungstag und Bekanntmachung des Hinweises auf die Patenterteilung: **25.10.2017**
(21) Anmeldenummer: 13726517.9
(22) Anmeldetag: 03.06.2013
(51) Int. Cl.: B65D 83/14, A61Q 17/04, A61K 8/04, A61K 8/06, A61K 8/39

(54) **NEUSTES KOSMETISCHES SONNENSCHUTZSPRAY**
LATEST COSMETIC SUNSCREEN SPRAY
NOUVEAU SPRAY COSMÉTIQUE SOLAIRE

(30) Priorität: 18.07.2012 DE 102012212548
(43) Veröffentlichungstag der Anmeldung: 27.05.2015
(73) Patentinhaber: Beiersdorf AG, 20253 Hamburg (DE)
(72) Erfinder: PETERSEN, Miriam, 22297 Hamburg (DE); KOCH, Petra, 22457 Hamburg (DE); MÖLLGAARD, Svenja Lena, 22337 Hamburg (DE)
(86) Internationale Anmeldenummer: PCT/EP2013/061351
(87) Internationale Veröffentlichungsnummer: WO 2014/012699

(56) Entgegenhaltungen:
- EP-A1- 2 380 577
- EP-A1- 2 407 163
- EP-A2- 0 904 773
- DATABASE GNPD [Online] MINTEL; 1. Juni 2011 (2011-06-01), Laboratorio Dermatológico Denenes: "Anti-Jellyfish Sunscreen SPF 50+", XP002728716, Database accession no. 1558554
- MEYER J ET AL: "A novel PEG-free emulsifier designed for formulating W/O lotions with a light skin feel", SOFW-JOURNAL SEIFEN, OELE, FETTE, WACHSE, VERLAG FUR CHEMISCHE INDUSTRIE, AUGSBURG, DE, Bd. 131, Nr. 11, 1. Januar 2005 (2005-01-01), Seiten 20-28, XP009104229, ISSN: 0942-7694
- "Isolan GPS", , 1. Mai 2003 (2003-05-01), Seiten 1-7, XP055135518, Gefunden im Internet: URL:http://www.quetzalquimica.com/images/D S_ISOLAN_GPS_e19-10-2007.pdf [gefunden am 2014-08-20]
- DATABASE GNPD [Online] MINTEL; 1. Juni 2011 (2011-06-01), DermaPharm: "Kids Sun Lotion SPF 30", XP002728717, Database accession no. 1558457

## Beschreibung

Die vorliegende Erfindung betrifft ein Aerosolspray mit einer kosmetischen W/O-Emulsion enthaltend Polyglyceryl-4 Diisostearate/Polyhydroxystearate/Sebacate sowie einen oder mehrere UV-Filter.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB-und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Sonnenschutzmittel werden in der Regel auf der Basis von O/W-Emulsionen (Öl-in-Wasser-Emulsionen) hergestellt, da O/W-Emulsionen aufgrund der äußeren wässrigen Phase eine angenehme Sensorik aufweisen und generell zu höheren Lichtschutzfaktoren führen (siehe Ärztl. Kosmetologie 7, 169-171 (1977)). Darüber hinaus lassen sich, im Gegensatz zur W/O-Emulsion, auf der Basis einer O/W-Emulsion dünnflüssige, gut versprühbare Zubereitungen herstellen. Nachteilig an O/W-Emulsionen ist jedoch der Umstand, dass diese Zubereitungen nicht wasserfest sind. Zur Erhöhung der Wasserfestigkeit müssen diesen Zubereitungen Filmbildner zugesetzt werden, die dann wiederum zu einer klebrig-unattraktiven Sensorik führen.

Es war daher die Aufgabe der vorliegenden Erfindung, die Nachteile des Standes der Technik zu beseitigen und ein Sonnenschutzmittel auf der Basis einer W/O-Emulsion (Wasser-in-Öl-Emulsion) zu entwickeln, welches einen hohen Lichtschutzfaktor aufweist und das gleichzeitig dünnflüssig und gut versprühbar ist.

Eine spezielle Form der Sonnenschutzmittel stellen die Sonnensprays dar. Hierbei unterscheidet der Fachmann zwischen sogenannten Pumpsprays, bei denen die Zubereitung mittels einer mechanischen Kolbenpumpe aus dem Vorratsbehältnis gefördert wird, sowie Zubereitungen, die mittels eines Treibgases aus einer Aerosoldose appliziert werden.

Bei diesen Aerosolsprays ist darauf zu achten, dass das Treibgas unter Druck in der Dose flüssig ist und damit nach dem Schütteln der Dose zum homogenen Bestandteil der lipophilen Phase der Emulsion wird. Durch die Lösung des Treibgases in der lipophilen Phase ändert sich deren Polarität und Lösungseigenschaften. Erst nach dem Entweichen der Zubereitung aus der Aerosoldose geht das Treibgas durch den Druckabfall in den gasförmigen Zustand über und entweicht wieder aus der lipophilen Phase. Um eine gute Versprühbarkeit der Zubereitung zu erreichen, muss deshalb dem Umstand Rechnung getragen werden, dass die lipophile Phase mit und ohne Treibgas eine homogene Beschaffenheit behält und es nicht in einem der beiden Zustandsformen (mit und ohne Treibgas) zu einer Ausfällung einzelner Bestandteile der lipophilen Phase, insbesondere von schwer löslichen UV-Filtern, Wachsen und Filmbildnern kommt. Dies ist jedoch bei den Zubereitungen des Standes der Technik regelmäßig der Fall. Dieser Effekt führt dann beispielsweise zur Verstopfung des Sprühkopfes der Aerosoldose.

Es war daher die Aufgabe der vorliegenden Erfindung, ein mit Hilfe eines Treibgases versprühbares Sonnenschutzmittel auf der Basis einer W/O-Emulsion zu entwickeln, bei dem es nicht zu Ausfällungen aus der lipophilen Phase kommt, wenn das Treibgas in der Phase gelöst oder nicht gelöst vorliegt.

Ein weiterer Nachteil an den Zubereitungen des Standes der Technik liegt in der schnellen Entmischung von durch Aufschütteln in der Zubereitung gelöstem Treibgas und Zubereitung. Im Zusammenspiel mit dem Treibgas kommt es bei den Stand der Technik-Rezepturen nach Schütteln zu einer vergleichsweisen schnellen Trennung von Bulk (= Zubereitung) und Gas. Dieser Effekt ist im Glasaerosolgefäß gut zu beobachten und ist für die Anwendung durch den Verbraucher nachteilig. Der Verbraucher muss entweder zeitnah erneut Schütteln oder verwendet ein Gemisch was anteilig zu viel Treibgas bzw. zu wenig Bulk enthält und benutzt unbewußt weniger Sonnenschutzmittel als eigentlich beabsichtigt. Nicht zuletzt führt die Verwendung von zu viel Treibgas dazu, dass sich später nicht mehr die gesamte Zubereitung aus dem Aerosolgefäß entnehmen lässt, da nicht mehr genügend Treibgas zur Entleerung vorhanden ist.

Es war daher die Aufgabe der vorliegenden Erfindung, die Nachteile des Standes der Technik zu beseitigen und ein Aerosol zu entwickeln, bei dem das Treibgas nach dem Schütteln unter Druck über einen längeren Zeitraum in der Zubereitung gelöst verbleibt.

Zur Herstellung von W/O-Emulsionen werden aus formulierungstechnischen Gründen gerne Emulgatoren mit Polyethylenglycol-Einheiten verwendet, da diese zu besonders stabilen Emulsionen führen und relativ unempfindlich sind gegenüber Änderungen in der Rezepturzusammensetzung. Derartige PEG-Emulgatoren sind jedoch bei den Verbrauchern zunehmend unerwünscht, weil diesen Verbindungen penetrationsförderne Eigenschaften unterstellt werden. Unabhängig davon, ob derartige Eigenschaften im Einzelfalle auch tatsächlich vorliegen, besteht eine Nachfrage nach PEG-freien W/O-Emusionen nicht zuletzt auch deshalb, weil deratige Zubereitungen bei der Bewertung durch die Zeitschrift "Öko-Test" zu Abwertungen führt. Nachteilig am Stande der Technik ist jedoch der Umstand, dass derartige Zubereitungen nicht besonders stabil und nur schwer versprühbar sind.

Es war daher die Aufgabe der vorliegenden Erfindung, eine stabile (d.h. temperatur- und lagerstabile), sensorisch ansprechende, "PEG"-freie, sprühbare Emulsion herzustellen.

Überraschend gelöst werden die Aufgaben durch ein kosmetisches Aerosolspray enthaltend eine W/O-Emulsion enthaltend
a) Polyglyceryl-4 Diisostearate/Polyhydroxystearate/Sebacate sowie
b) einen oder mehrere UV-Filter,
dadurch gekennzeichnet, dass die W/O-Emulsion mit einem Treibgas versetzt ist, wobei das Treibgas gewählt wird aus Propan, n-Butan, Isobutan und deren Mischungen.

Zwar kennt der Stand der Technik die EP 1500427, EP 2088988, WO 2006/134886, US 7829106, EP 1549281 und DE 102010050774, sowie EP 2380577, EP 0904773, EP 2407163, die Einträge in die GNPD-Datenbank (Mintel) Nummer 1558554, 1558457, J. Meyer et al. SÖFW-Journal Seiten 20-28, Band 131, Nr. 11, Januar 2005 und URL:http.//www.quetzalquimica.com/images/DS_ISOLAN_GPS_e19-10-2007.pdf, doch konnten diese Schriften nicht den Weg zur vorliegenden Erfindung weisen.

Die Formulierungen "erfindungsgemäße Zubereitung" etc. beziehen sich im Rahmen der vorliegenden Offenbarung auf die erfindungsgemäße W/O-Emulsion, auch wenn dies nicht explizit erwähnt ist.

Alle Konzentrationsangaben im Rahmen der vorliegenden Offenbarung beziehen sich, falls es nicht explizit angegeben ist, auf eine Zubereitung ohne Treibgas.

In der erfindungsgemäß vorteilhaften Ausführungsform der PEG-freien Zubereitungen sind die Zubereitungen überraschend temperatur- und lagerstabil und zeichnen sich dabei durch eine überraschend leichte, nicht klebrige Sensorik aus.

Neben der klassisch bekannten "Lagerstabilität" bei unterschiedlichen Temperaturen, bezieht sich die Stabilität bei erfindungsgemäßen Zubereitungen zusätzlich auf die langsamere Entmischung nach Schütteln.

Aerosol-Produkte werden vor Anwendung geschüttelt. Nach dem Stand der Technik zeigen die geschüttelten Zubereitungen, bei denen das Treibgas dann in der Ölphase gelöst ist, häufig bei Raumtemperatur eine Ölabscheidung. Erfindungsgemäße Zubereitungen zeigen hingegen deutlich längere Entmischungszeiten nach Schütteln. Dabei kann "länger" in diesem Zusammenhang mehr als 3 Stunden bedeuten. In besonders bevorzugen Ausführungsformen zeigten die Zubereitungen eine Ölabscheidung bei Raumtemperatur von mehr als einem Monat.

Die sensorischen Eigenschaften der erfindungsgemäßen Zubereitungen werden von Anwendern als angenehm und nicht klebrig beurteilt.

Besonders überraschend war nicht zuletzt der Umstand, dass der Lichtschutzfaktor (SPF) der erfindungsgemäßen Zubereitung nach dem Baden ansteigt, wenn der Anwender die erfindungsgemäße Zubereitung auf die Haut aufgetragen hat und anschließend mit Wasser in Kontakt kommt.

Es ist erfindungsgemäß bevorzugt, wenn die Zubereitung Polyglyceryl-4 Diisostearate/Polyhydroxystearate/Sebacate in einer Konzentration von 0,5 bis 5 Gewichts%, bezogen auf das Gesamtgewicht der Zubereitung, enthält.

Es ist erfindungsgemäß besonders bevorzugt, wenn die Zubereitung Polyglyceryl-4 Diisostearate/Polyhydroxystearate/Sebacate in einer Konzentration von 2 bis 4 Gewichts-%, bezogen auf das Gesamtgewicht der Zubereitung, enthält.

Es ist erfindungsgemäß vorteilhaft, wenn die Zubereitung zusätzlich Diisostearoyl Polyglyceryl-3 Dimer Dioleate enthält.

In einem solchen Fall ist es bevorzugt im Sinne der vorliegenden Erfindung, wenn die Zubereitung Diisostearoyl Polyglyceryl-3 Dimer Dioleate in einer Konzentration von 0,5 bis 5 Gewichts-%, bezogen auf das Gesamtgewicht der Zubereitung, enthält.

Es ist erfindungsgemäß vorteilhaft, wenn die Zubereitung mit Magnesiumsulfat oder Natriumchlorid in einer Konzentration von 0,1 bis 3 Gewichts-%, bezogen auf das Gesamtgewicht der Zubereitung stabilisiert ist. Dabei ist der Einsatz von Magnesiumsulfat erfindungsgemäß bevorzugt.

Es ist erfindungsgemäß vorteilhaft, wenn die erfindungsgemäße Zubereitung einen oder mehrere UV-Filter enthält, gewählt aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornyliden-methyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 4-(tert.-Butyl)-4'-methoxydibenzoylmethan; 2-Ethylhexyl-2-cyano-3,3-diphenylacrylat; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester; Homomenthylsalicylat; 2-Ethylhexyl-2-hydroxybenzoat; Dimethicodiethylbenzalmalonat; 3-(4-(2,2-bis Ethoxycarbonylvinyl)-phe-noxy)propenyl)-methoxysiloxan / Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoesäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4-Bis-{[4-(2-ethyl-hexyloxy)-2-hydroxy]-phenyl}-6-(4-methoxyphenyl)-1,3,5-triazin; 2,4,6-Tribiphenyl-4-yl-1,3,5-triazin; Merocyanine; Titandioxid; Zinkoxid.

Erfindungsgemäß vorteilhaft enthält die erfindungsgemäße Zubereitung UV-Filter in einer Menge von 1 bis 30 Gewichts-%, bezogen auf das Gesamtgewicht der Zubereitung.

Erfindungsgemäß bevorzugt ist die erfindungsgemäße Zubereitung frei ist von 3-(4-Methylbenzyliden)campher.

Erfindungsgemäß bevorzugt ist die erfindungsgemäße Zubereitung frei ist von Benzophenone-3 bzw. Benzophenone-4.

Es ist erfindungsgemäß vorteilhaft, wenn die Zubereitung 4-(tert.-Butyl)-4'-methoxydiben-zoylmethan und/oder 2-(4'-Diethylamino-2'-hydoxybenzoyl)-benzoesäurehexylester enthält.

Erfindungsgemäß besonders bevorzugt ist dabei ein Gehalt an 4-(tert.-Butyl)-4'-methoxydi-benzoylmethan.

4-(tert.-Butyl)-4'-methoxydibenzoylmethan und 2-(4'-Diethylamino-2'-hydoxybenzoyl)-benzoesäurehexylester werden dabei erfindungsgemäß vorteilhaft einzeln oder als Mischung (bevorzugt einzeln) in einer Gesamtkonzentration von 1 bis 10 Gewichts-%, bezogen auf das Gesamtgewicht der Zubereitung eingesetzt. Erfindungsgemäß bevorzugt wird ein Gehalt von 3,5 bis 5 Gewichts-% und besonders bevorzugt 4,5%, bezogen auf das Gesamtgewicht der Zubereitung, eingesetzt.

Es ist erfindungsgemäß bevorzugt, wenn die erfindungsgemäße Zubereitung einen oder mehrere UV-Filter gewählt aus der Gruppe der Verbindungen der Triazine enthält.

In einem solchen Falle ist es erfindungsgemäß besonders bevorzugt, wenn die erfindungsgemäße Zubereitung 2,4-Bis-{[4-(2-ethyl-hexyloxy)-2-hydroxy]-phenyl}-6-(4-methoxyphenyl)-1,3,5-triazin enthält.

Enthält die erfindungsgemäße Zubereitung 2,4-Bis-{[4-(2-ethyl-hexyloxy)-2-hydroxy]-phenyl)-6-(4-methoxyphenyl)-1,3,5-triazin, so wird dieser UV-Filter erfindungsgemäß vorteilhaft in einer Konzentration von 0,5 bis 10 Gewichts-%, bezogen auf das Gesamtgewicht der Zubereitung, eingesetzt.

Es ist erfindungsgemäß besonders bevorzugt, wenn die Zubereitung Octocrylen enthält.

Enthält die Zubereitung Octocrylen, so wird diese Verbindung erfindungsgemäß vorteilhaft in einer Konzentration von 1 bis 10 Gewichts-%, bezogen auf das Gesamtgewicht der Zubereitung eingesetzt.

Es ist erfindungsgemäß, wenn die erfindungsgemäße Zubereitung mit einem Treibgas versetzt wird. Dies geschieht erfindungsgemäß bevorzugt in einer Aerosoldose. Mit diesem Treibgas kann die Zubereitung dann erfindungsgemäß vorteilhaft versprüht werden. Diese Zubereitungen weisen dann ein überraschend leichtes sensorisches Gefühl auf der Haut auf.

Erfindungsgemäß besonders bevorzugt, ist eine Mischung des Treibgases aus 60 Gewichts-% n-Butan, 20 Gewichts-% Isobutan und 20 Gewichts-% Propan (Druckstufe 2,7 bar) oder 72 Gewichts-% Isobutan +23 Gewichts-% Propan + 5 Gewichts-% n-Butan (Druckstufe 3,5 bar), wobei Schwankungen von jeweils 10% noch als erfindungsgemäß gelten.

Wird die erfindungsgsgemäße Zubereitung mit Treibgas versetzt, so ist es erfindungsgemäß vorteilhaft, wenn das Verhältnis von Zubereitung zu Treibgas von 60-80 Gewichts-% Zubereitung zu 40-20 Gewichts-% Treibgas .beträgt.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile® (CAS-Nr. 7631-86-9), Talkum, Lauroyl Lysine und Acrylonitrile-methacrylonitrile-methyl-methacrylate.

Erfindungsgemäß bevorzugt ist es, wenn die Zubereitung Füllstoffe, insbesondere Polyethylen, Nylon, natürliche oder modifizierte Stärken wie Tapiokastärke und/oder Silikate wie beispielsweise Talkum enthält.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl wie Isopropanol, Diole oder Polyole niedriger C-Zahl sowie deren Ether, vorzugsweise Propylenglykol, 2-Methylpropan-1,3-diol, Pentan-1,2-diol, Hexan-1,2-diol, Octan-1,2-diol, Decan-1,2-diol, Glycerin, Ethylenglykol, Ethylenglykolmonoethyl-oder-monobutylether, Propylenglykolmonomethyl, -monoethyl- oder -monobutylether, Diethylenglykolmonomethyl-oder -monoethylether und analoge Produkte, Schaumstabilisatoren, Elektrolyte, etc.. Die erfindungsgemäße Zubereitung kann vorteilhaft Konsistenzbildner (Gelbildner, Verdickungsmittel) wie beispielsweise Polyacrylate (auch quervernetzt) oder Cellulosederivate (beispielsweise Hydroxyethylcellulose) oder andere enthalten.

Erfindungsgemäß bevorzugt ist es, wenn die erfindungsgemäße Zubereitung dadurch gekennzeichnet ist, dass die Zubereitung Propylenglycol, Butylenglycol, 2-Methylpropan-1,3-diol, 1,2-Pentandiol, 1,2-Hexandiol, 1,2-Octandiol und/oder 1,2-Decandiol enthält. Dabei werden diese Diole/Glycole erfindungsgemäß vorteilhaft in einer Gesamtmenge von 0,1 bis 3 Gewichts-% bezogen auf das Gesamtgewicht der Zubereitung, eingesetzt.

Es ist erfindungsgemäß bevorzugt, wenn die Zubereitung Xanthangummi enthält.

Selbstverständlich kann die Zubereitung mit den üblichen, in der Kosmetik verwendeten Konservierungsmitteln konserviert werden.

Dabei ist es erfindungsgemäß bevorzugt, wenn die Zubereitung Phenoxyethanol enthält.

Darüber hinaus ist es erfindungsgemäß vorteilhaft, wenn die erfindungsgemäße Zubereitung Kaliumsorbat enthält, das erfindungsgemäß bevorzugt in einer Konzentration von 0,05 bis 0,2 Gewichts-%, bezogen auf das Gesamtgewicht der Zubereitung, eingesetzt wird.

Darüber hinaus ist es erfindungsgemäß bevorzugt, wenn die Zubereitung frei ist von Parabenen.

Erfindungsgemäß bevorzugt ist die erfindungsgemäße Zubereitung frei ist von Polyethylenglycolen.

Die Ölphase der erfindungsgemäßen Zubereitung wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Jojobaöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Phenethylbenzoat, 2-Phenylethylbenzoat, Isopropyl Lauroyl Sarkosinat, Phenyl Trimethicon, Cyclomethicon, Dibutyladipat, Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol* CC bei der Fa. Cognis erhältliche.

Es ist ferner vorteilhaft, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Tridecylsalicylat (welches unter der Handelsbezeichnung Cosmacol ESI bei der Fa. Sasol erhältlich ist), C12-C15 Alkylsalicylat (unter der Handelsbezeichnung Dermol NS bei der Fa. Alzo erhältlich), Hexadecylbenzoat und Butyloctylbenzoat und Gemische davon (*Hallstar AB*)*.*

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene, C13-16 Isoparaffin und Isohexadecan. Unter den Polyolefinen sind Polydecene die bevorzugten Substanzen.

Es ist erfindungsgemäß vorteilhaft, wenn die erfindungsgemäße Zubereitung einen oder mehrere Wirkstoffe gewählt aus der Gruppe der Verbindungen Gylcyrrhetinsäure, Harnstoff, Arctiin, alpha-Liponsäure, Folsäure, Phytoen, D-Biotin, Coenzym Q10, alpha-Glucosylrutin, Carnitin, Carnosin, Coffein, natürliche und/oder synthetische Isoflavonoide, Glycerylglucose, Kreatin, Kreatinin, Taurin, Tocopherol, Tocopherolacetat, ß-Alanin und/oder Licochalcon A, enthält.

Erfindungsgemäß vorteilhaft wird die erfindungsgemäße Zubereitung in einer Treibgasdose, enthaltend die kosmetische Zubereitung, dargereicht.

Dabei ist es erfindungsgemäß bevorzugt, wenn die Dose eine Aluminiumdose ist, die auf der Innenseite mit einem Schutzlack beschichtet ist.

In einem solchen Falle ist es erfindungsgemäß vorteilhaft, wenn der Schutzlack ein Polyamid-Imid-Lack oder ein Pulverlack ist.

Erfindungsgemäß vorteilhafte Polyamid-Imid-(PAM)- Lacke sind beispielsweise PPG8460-303A Gold PAM-2-C Internal Liner 8460N von der Firma HOBA.

Ein erfindungsgemäß vorteilhafter Pulverlack ist beispielsweise der Epoxy Pulverlack Drylac 069/10103 von der Firma Tiger.

Es ist erfindungsgemäß vorteilhaft, wenn das Ventil der Treibgasdose im Sprühkopf ein Ariane Kugelventil ist. Ein Beispiel hierfür sind Kugelventile der Firma Aptar.

Das Ventil hat erfindungsgemäß vorteilhaft einen Aluteller, der lackiert ist mit z.B. Micoflex-Lack (L6X392L/5 basecoat mit L3E692S/5 topcoat oder L6X392L/6 basecoat mit L3E692S/6 topcoat) von Valspar oder Heliotherm Schutzlack VP 16506.

Die Kegelbohrung kann erfindungsgemäß vorteilhaft 1x 0,32 mm oder 1x 0,40mm oder 1x 0,5mm betragen. Mögliche Tellerdichtungen sind z.B. Nitril B 175, Nitril BA 7402, Nitril KA 6712, Nitril Buna 1602, Chlorbutyl CA 6600, Neoprene NA 7202, Butyl U 133, Buna 5252 6.

Darüber hinaus sind solche Treibbgasdosen erfindungsgemäß vorteilhaft dadurch gekennzeichnet, dass als Sprühkopf z.B. der WS 25 von Aptar eingesetzt wird, mit folgenden Düsen: WAZ 4833, WAZ 5118, WAZ 3832 oder WAZ AT 5034.

Die erfindungsgemäßen Zubereitungen werden erfindungsgemäß bevorzugt als Sonnenschutzmittel verwendet. Darüber hinaus ist ihr Einsatz als Tagespflegeprodukt erfindungsgemäß.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

Die Zubereitungen (=Bulk) wurden in eine Aerosoldose abgefüllt, die auf der Innenseite mit einem Polyamid-Imid-Lack (PPG8460-303A Gold PAM-2-C Internal Liner 8460N von der Firma HOBA) oder mit dem Epoxy Pulverlack Drylac 069/10103 von der Firma Tiger beschichtet war und mit der Treibgasmischung versetzt.

Als Sprühkopf wurde Sprühkopf WS 25 von Aptar eingesetzt wird, mit folgenden Düsen: WAZ 4833, WAZ 5118, WAZ 3832 oder WAZ AT 5034.

Als Ventil der Treibgasdose im Sprühkopf wurde ein Ariane Kugelventil ist der Firma Aptar verwendet, welches einen lackierten Aluminiumteller aufwies.

Dabei wurden Kegelbohrungen von 1x 0,32 mm oder 1x 0,40mm oder 1x 0,5mm verwendet.

| **Beipielrezeptur** | **1** | **2** | **3** | **4** | **5** |
|---|---|---|---|---|---|
| Piroctone Olamine | 0,05 | | | | |
| C13-16 Isoparaffin | 12 | | | | 12 |
| Butylene Glycol Dicaprylate/Dicaprate | 8,5 | 8,5 | 8,5 | 8,5 | 8,5 |
| Isohexadecane | | 12 | | | |
| Isopropyl Stearate | | | 12 | 12 | |
| Polyglyceryl-4 Diisostearate/Polyhydroxystearate/Sebacate | 3 | 3 | 3 | 3 | 3 |
| Parfum | 0,3 | 0,3 | 0,3 | 0,3 | 0,3 |
| Glycerin | 4,95 | 4,95 | 4,95 | 4,95 | 4,95 |
| Sodium Hydroxide | 0,225 | 0,225 | 0,225 | | |
| Phenoxyethanol | 0,5 | 0,5 | 0,5 | 0,5 | 0,5 |
| Ethylparaben | | 0,1 | 0,1 | 0,1 | 0,1 |
| Methylparaben | | 0,3 | 0,3 | 0,3 | 0,3 |
| Aqua | ad 100 | ad 100 | ad 100 | ad 100 | ad 100 |
| Magnesium Sulfate | 0,7 | 0,7 | 0,7 | 0,7 | 0,7 |
| Trisodium EDTA | 0,2 | 0,2 | 0,2 | 0,2 | 0,2 |
| Octocrylene | 7 | 7 | 7 | 9,5 | 9 |
| Ethylhexyl Salicylate | 4,5 | 4,5 | 4,5 | 4,5 | 4,5 |
| Bis-Ethylhexyloxyphenol Methoxyphenyl Triazine | 2,5 | 2,5 | 2,5 | 2 | 2 |
| Butyl Methoxydibenzoylmethane | 4,5 | 4,5 | 4,5 | 4,5 | 4,5 |
| Phenylbenzimidazole Sulfonic Acid | 1,5 | 1,5 | 1,5 | | |
| Homosalate | | | | 9,5 | 9 |
| Polysilicone-15 | | | | | 1 |

Der Ansatz setzt sich zusammen aus 75% Bulk und 25 % Treibgas (60% Butane + 20% Isobutane + 20% Propane, Druckstufe 2,7 bar)

| **Beispielrezeptur** | **6** | **7** | **8** | **9** | **10** |
|---|---|---|---|---|---|
| C13-16 Isoparaffin | 12 | 12 | 12 | 12 | |
| Butylene Glycol Dicaprylate/Dicaprate | 8,5 | 8,5 | 8,5 | 8,5 | 8,5 |
| Isopropyl Palmitate | | | | | 12 |
| Polyglyceryl-4 Diisostearate/Polyhydroxystearate/Sebacate | 3,5 | 4 | 3,5 | 4 | 3 |
| Parfum | 0,3 | 0,3 | 0,3 | 0,3 | 0,3 |
| Glycerin | 4,95 | 4,95 | 4,95 | 4,95 | 4,95 |
| Sodium Hydroxide | 0,225 | 0,225 | | | 0,225 |
| Ethylparaben | 0,1 | 0,1 | 0,1 | 0,1 | 0,1 |
| Methylparaben | 0,3 | 0,3 | 0,3 | 0,3 | 0,3 |
| Phenoxyethanol | 0,5 | 0,5 | 0,5 | 0,5 | 0,5 |
| Aqua | ad 100 | ad 100 | ad 100 | ad 100 | ad 100 |
| Magnesium Sulfate | 0,7 | 0,7 | 0,7 | 0,7 | 0,7 |
| Trisodium EDTA | 0,2 | 0,2 | 0,2 | 0,2 | 0,2 |
| Octocrylene | 7 | 7 | 9,5 | 9,5 | 7 |
| Ethylhexyl Salicylate | 4,5 | 4,5 | 4,5 | 4,5 | 4,5 |
| Bis-Ethylhexyloxyphenol Methoxyphenyl Triazine | 2,5 | 2,5 | 2 | 2 | 2,5 |
| Butyl Methoxydibenzoylmethane | 4,5 | 4,5 | 4,5 | 4,5 | 4,5 |
| Phenylbenzimidazole Sulfonic Acid | 1,5 | 1,5 | | | 1,5 |
| Homosalate | | | 9,5 | 9,5 | |

Der Ansatz setzt sich zusammen aus 75% Bulk und 25% Treibgas (72% Isobutan +23% Propan + 5% n-Butan, Druckstufe 3,5 bar)

| **Beispielrezeptur** | **11** | **12** | **13** | **14** | **15** |
|---|---|---|---|---|---|
| Piroctone Olamine | | | 0,05 | 0,05 | |
| Panthenol | | | | | 1,078 |
| Cl 42090 | | | | | 0,001 |
| Isopropyl Palmitate | 12 | | | | |
| Butylene Glycol Dicaprylate/Dicaprate | 8,5 | 8,5 | 8,5 | 8,5 | 8,5 |
| C13-16 Isoparaffin | | 12 | 12 | 12 | |
| Isopropyl Stearate | | | | | 12 |
| Polyglyceryl-4 Diisostearate/Polyhydroxystearate/Sebacate | 3 | 3 | 3 | 3 | 3 |
| Parfum | 0,3 | 0,3 | 0,3 | 0,3 | 0,3 |
| Glycerin | 4,95 | 4,95 | 4,95 | 4,95 | 4,95 |
| Sodium Hydroxide | | 0,225 | 0,225 | | |
| Ethylparaben | 0,1 | 0,1 | 0,1 | 0,1 | 0,1 |
| Methylparaben | 0,3 | 0,3 | 0,3 | 0,3 | 0,3 |
| Phenoxyethanol | 0,5 | 0,5 | 0,5 | 0,5 | 0,5 |
| Aqua | ad 100 | ad 100 | ad 100 | ad 100 | ad 100 |
| Trisodium EDTA | 0,2 | 0,2 | 0,2 | 0,2 | 0,2 |
| Magnesium Sulfate | 0,7 | 1,5 | 0,7 | 0,7 | 0,7 |
| Homosalate | 9,5 | | 9,5 | 9,5 | 9,5 |
| Octocrylene | 9,5 | 7 | 9,5 | 9,5 | 9,5 |
| Ethylhexyl Salicylate | 4,5 | 4,5 | 4,5 | 4,5 | 4,5 |
| Bis-Ethylhexyloxyphenol Methoxyphenyl Triazine | 2 | 2,5 | 4 | 4 | 2 |
| Butyl Methoxydibenzoylmethane | 4,5 | 4,5 | 4,5 | 4,5 | 4,5 |
| Phenylbenzimidazole Sulfonic Acid | | 1,5 | 1,5 | | |
| Ethylhexyl Methoxycinnamate + BHT | | | | 0,5 | |
| Diethylhexyl Butamido Triazone | | | | 2 | |

Der Ansatz setzt sich zusammen aus 75% Bulk und 25 % Treibgas (60% Butane + 20% Isobutane + 20% Propane, Druckstufe 2,7 bar)

| **Beispielrezeptur** | **16** | **17** | **18** | **19** | **20** |
|---|---|---|---|---|---|
| Ethylparaben | | 0,100 | | | |
| Methylparaben | | 0,300 | | | |
| Aqua | ad 100 | ad 100 | ad 100 | ad 100 | ad 100 |
| Magnesium Sulfate | 0,700 | 0,700 | 0,700 | 0,700 | |
| Trisodium EDTA | 0,200 | 0,200 | 0,200 | 0,200 | 0,200 |
| Phenoxyethanol | 0,800 | 0,500 | 0,500 | 0,500 | 0,500 |
| Butyl Methoxydibenzoylmethane | 4,500 | 4,500 | 4,500 | 4,500 | 4,500 |
| Phenylbenzimidazole Sulfonic Acid | 1,500 | 1,500 | 1,500 | 1,500 | 1,500 |
| Glycerin | 4,950 | 4,950 | 4,950 | 4,950 | 4,950 |
| Sodium Hydroxide | 0,255 | 0,255 | 0,225 | 0,225 | 0,216 |
| Ethylhexyl Salicylate | 4,500 | 4,500 | 4,500 | 4,500 | 4,500 |
| Piroctone Olamine | | | 0,050 | 0,050 | 0,050 |
| Potassium Chloride | | | | | 3,000 |
| Octocrylene | 7,000 | 7,000 | 7,000 | 7,000 | 7,000 |
| Bis-Ethylhexyloxyphenol Methoxyphenyl Triazine | 2,500 | 2,500 | 2,500 | 2,500 | 2,500 |
| Butylene Glycol Dicaprylate/Dicaprate | 8,500 | 8,500 | 8,500 | 8,500 | 8,500 |
| C13-16 Isoparaffin | 12,000 | 12,000 | 12,000 | 12,000 | 12,000 |
| Methylpropanediol | | 2,000 | | | |
| 1,2-Hexanediol | 1,000 | | | | |
| Polyglyceryl-4 Diisostearate/Polyhydroxystearate/Sebacate | 3,000 | 3,000 | 2,000 | 2,750 | 3,000 |
| Parfum | 0,300 | 0,300 | 0,300 | 0,300 | 0,300 |

Der Ansatz setzt sich zusammen aus 75% Bulk und 25% Treibgas (72% Isobutan +23% Propan + 5% n-Butan, Druckstufe 3,5 bar)

| **Beispielrezeptur** | **21** | **22** | **23** | **24** | **25** |
|---|---|---|---|---|---|
| Ethylparaben | 0,100 | 0,000 | 0,100 | 0,100 | 0,100 |
| Methylparaben | 0,300 | 0,000 | 0,300 | 0,300 | 0,300 |
| Aqua | ad 100 | ad 100 | ad 100 | ad 100 | ad 100 |
| Trisodium EDTA | 0,200 | 0,200 | 0,200 | 0,200 | 0,200 |
| Phenoxyethanol | 0,500 | 0,500 | 0,500 | 0,500 | 0,500 |
| Butyl Methoxydibenzoylmethane | 4,500 | 4,500 | 4,500 | 4,500 | 4,500 |
| Phenylbenzimidazole Sulfonic Acid | 1,500 | | 1,500 | | |
| Glycerin | 4,950 | 4,950 | 4,950 | 4,950 | 4,950 |
| Sodium Hydroxide | 0,400 | | 0,216 | | |
| Isopropyl Stearate | | 12,000 | | 12,000 | 14,000 |
| Ethylhexyl Salicylate | 4,500 | 4,500 | 4,500 | 4,500 | 4,500 |
| Sodium Chloride | 3,000 | 3,000 | 3,000 | 3,000 | 3,000 |
| Piroctone Olamine | | 0,050 | | | |
| Octocrylene | 7,000 | 9,500 | 7,000 | 9,500 | 9,500 |
| Homosalate | | 9,500 | | 9,500 | 9,500 |
| Bis-Ethylhexyloxyphenol Methoxyphenyl Triazine | 2,500 | 2,000 | 2,500 | 2,000 | 2,000 |
| Butylene Glycol Dicaprylate/Dicaprate | 8,500 | 10,000 | 14,000 | 14,000 | 14,000 |
| Microcrystalline Cellulose + Cellulose Gum | 0,100 | | | | |
| C13-16 Isoparaffin | 12,000 | | 12,000 | | |
| Polyglyceryl-4 Diisostearate/Polyhydroxystearate/Sebacate | 3,000 | 3,000 | 3,000 | 2,500 | 2,500 |
| Parfum | 0,300 | 0,300 | 0,300 | 0,300 | 0,300 |

Der Ansatz setzt sich zusammen aus 75% Bulk und 25 % Treibgas (60% Butane + 20% Isobutane + 20% Propane, Druckstufe 2,7 bar)

| **Beispielrezeptur** | **26** | **27** | **28** | **29** | **30** |
|---|---|---|---|---|---|
| Ethylparaben | 0,100 | 0,100 | 0,100 | 0,100 | 0,100 |
| Methylparaben | 0,300 | 0,300 | 0,300 | 0,300 | 0,300 |
| Aqua | ad 100 | ad 100 | ad 100 | ad 100 | ad 100 |
| Magnesium Sulfate | | | 0,700 | 0,700 | 0,700 |
| Trisodium EDTA | 0,200 | 0,200 | 0,200 | 0,200 | 0,200 |
| Phenoxyethanol | 0,500 | 0,500 | 0,500 | 0,500 | 0,500 |
| Panthenol | | 1,078 | | | |
| Butyl Methoxydibenzoylmethane | 4,500 | 4,500 | 4,500 | 4,500 | 4,500 |
| Phenylbenzimidazole Sulfonic Acid | 1,500 | | | 1,500 | |
| Glycerin | 4,950 | 4,950 | 4,950 | 4,950 | 4,950 |
| Sodium Hydroxide | 0,216 | | | 0,216 | |
| Butylene Glycol | 5,000 | | | | |
| Isopropyl Stearate | | 12,000 | 12,000 | | 12,000 |
| Ethylhexyl Salicylate | 4,500 | 4,500 | 4,500 | 4,500 | 4,500 |
| Sodium Chloride | 3,000 | 3,000 | | | |
| Cl 42090 | | 0,001 | | | |
| Octocrylene | 7,000 | 9,500 | 9,500 | 7,000 | 9,500 |
| Homosalate | | 9,500 | 9,500 | 9,500 | 9,500 |
| Bis-Ethylhexyloxyphenol Methoxyphenyl Triazine | 2,500 | 2,000 | 5,000 | 2,500 | 2,500 |
| Butylene Glycol Dicaprylate/Dicaprate | 14,000 | 8,500 | 8,500 | 8,500 | 8,500 |
| C13-16 Isoparaffin | 12,000 | | | 12,000 | |
| Polyglyceryl-4 Diisostearate/Polyhydroxystearate/Sebacate | 2,500 | 3,000 | 3,000 | 3,000 | 3,000 |
| Parfum | 0,300 | 0,300 | 0,300 | 0,300 | 0,300 |

Der Ansatz setzt sich zusammen aus 75% Bulk und 25% Treibgas (72% Isobutan +23% Propan + 5% n-Butan, Druckstufe 3,5 bar)

| **Beispielrezeptur** | **31** | **32** | **33** | **34** | **35** |
|---|---|---|---|---|---|
| Ethylparaben | 0,100 | 0,100 | 0,100 | 0,100 | 0,100 |
| Methylparaben | 0,300 | 0,300 | 0,300 | 0,300 | 0,300 |
| Aqua | ad 100 | ad 100 | ad 100 | ad 100 | ad 100 |
| Magnesium Sulfate | 0,700 | 0,700 | 0,700 | 0,700 | 0,700 |
| Trisodium EDTA | 0,200 | 0,200 | 0,200 | 0,200 | 0,200 |
| Phenoxyethanol | 0,500 | 0,500 | 0,500 | 0,500 | 0,500 |
| Panthenol | | | | | 1,078 |
| Butyl Methoxydibenzoylmethane | 4,500 | 4,500 | 4,500 | 4,500 | 4,500 |
| Glycerin | 4,950 | 4,950 | 4,950 | 4,950 | 4,950 |
| Isopropyl Stearate | 12,000 | 12,000 | 12,000 | 12,000 | 12,000 |
| Ethylhexyl Salicylate | 4,500 | 4,500 | 4,500 | 4,500 | 4,500 |
| Titanium Dioxide (nano) + Trimethoxycaprylylsilane | | | 2,000 | | |
| Cl 42090 | | | | | 0,001 |
| Octocrylene | 9,500 | 9,500 | 9,500 | 9,500 | 9,500 |
| Homosalate | 9,500 | 9,500 | 9,500 | 9,500 | 9,500 |
| Polysilicone-15 | | 2,000 | | | |
| Bis-Ethylhexyloxyphenol Methoxyphenyl Triazine | 3,000 | 4,000 | 4,000 | 4,000 | 2,000 |
| Butylene Glycol Dicaprylate/Dicaprate | 8,500 | 8,500 | 8,500 | 8,500 | 8,500 |
| Diisostearoyl Polyglyceryl-3 Dimer Dilinoleate | | | | | 0,500 |
| Polyglyceryl-4 Diisostearate/Polyhydroxystearate/Sebacate | 3,000 | 3,000 | 3,000 | 3,000 | 3,000 |
| Parfum | 0,300 | 0,300 | 0,300 | 0,300 | 0,300 |

Der Ansatz setzt sich zusammen aus 75% Bulk und 25 % Treibgas (60% Butane + 20% Isobutane + 20% Propane, Druckstufe 2,7 bar)

| **Beispielrezeptur** | **36** | **37** | **38** | **39** | **40** |
|---|---|---|---|---|---|
| Ethylparaben | 0,100 | 0,100 | 0,100 | 0,100 | 0,100 |
| Methylparaben | 0,300 | 0,300 | 0,300 | 0,300 | 0,300 |
| Aqua | ad 100 | ad 100 | ad 100 | ad 100 | ad 100 |
| Aluminum Stearates | | | | | 0,150 |
| Magnesium Sulfate | 0,700 | 0,700 | 0,700 | 0,700 | 0,700 |
| Magnesium Stearate | | | 0,200 | 0,300 | 0,150 |
| Trisodium EDTA | 0,200 | 0,200 | 0,200 | 0,200 | 0,200 |
| Phenoxyethanol | 0,500 | 0,500 | 0,500 | 0,500 | 0,500 |
| Panthenol | 1,078 | 1,078 | 1,078 | 1,078 | 1,078 |
| Butyl Methoxydibenzoylmethane | 4,500 | 4,500 | 4,500 | 4,500 | 4,500 |
| Glycerin | 4,950 | 4,950 | 4,950 | 4,950 | 5,000 |
| Isopropyl Stearate | 12,000 | 12,000 | 12,000 | 12,000 | 12,000 |
| Ethylhexyl Salicylate | 4,500 | 4,500 | 4,500 | 4,500 | 4,500 |
| Cl 42090 | 0,001 | 0,001 | 0,001 | 0,001 | 0,001 |
| Octocrylene | 9,500 | 9,500 | 9,500 | 9,500 | 9,500 |
| Homosalate | 9,500 | 9,500 | 9,500 | 9,500 | 9,500 |
| Bis-Ethylhexyloxyphenol Methoxyphenyl Triazine | 2,000 | 2,000 | 2,000 | 2,000 | 2,000 |
| Butylene Glycol Dicaprylate/Dicaprate | 8,500 | 8,500 | 8,500 | 8,500 | 8,500 |
| Diisostearoyl Polyglyceryl-3 Dimer Dilinoleate | 0,750 | 1,250 | | | |
| Polyglyceryl-4 Diisostearate/Polyhydroxystearate/Sebacate | 3,000 | 3,000 | 3,000 | 3,000 | 3,000 |
| Parfum | 0,300 | 0,300 | 0,300 | 0,300 | 0,300 |

Der Ansatz setzt sich zusammen aus 75% Bulk und 25% Treibgas (72% Isobutan +23% Propan + 5% n-Butan, Druckstufe 3,5 bar)

| **Beispielrezeptur** | **41** | **42** | **43** | **44** | **45** |
|---|---|---|---|---|---|
| Ethylparaben | 0,100 | 0,100 | 0,100 | 0,100 | 0,100 |
| Methylparaben | 0,300 | 0,300 | 0,300 | 0,300 | 0,300 |
| Aqua | ad 100 | ad 100 | ad 100 | ad 100 | ad 100 |
| Magnesium Sulfate | 0,700 | 0,700 | 0,700 | 0,700 | 0,700 |
| Trisodium EDTA | 0,200 | 0,200 | 0,200 | 0,200 | 0,200 |
| Phenoxyethanol | 0,500 | 0,500 | 0,500 | 0,500 | 0,500 |
| Butyl Methoxydibenzoylmethane | 4,500 | 4,500 | 4,500 | 4,500 | 4,500 |
| VP/Hexadecene Copolymer | | 0,500 | | | |
| Glycerin | 4,950 | 4,950 | 4,950 | 4,950 | 4,950 |
| Isopropyl Stearate | 12,000 | 12,000 | 12,000 | 12,000 | 12,000 |
| Ethylhexyl Salicylate | 4,500 | 4,500 | 4,500 | 4,500 | 4,500 |
| Silica Dimethyl Silylate | 0,500 | | | | |
| Ethylhexylglycerin | | | | | 0,495 |
| Octocrylene | 9,500 | 9,500 | 9,500 | 9,500 | 9,500 |
| Homosalate | 9,500 | 9,500 | 9,500 | 9,500 | 9,500 |
| Bis-Ethylhexyloxyphenol Methoxyphenyl Triazine | 2,000 | 2,000 | 2,000 | 2,000 | 2,000 |
| Butylene Glycol Dicaprylate/Dicaprate | 8,500 | 8,500 | 8,500 | 8,500 | 8,500 |
| Methylpropanediol | | | | 2,000 | |
| 1,2-Hexanediol | | | 0,500 | | |
| Polyglyceryl-4 Diisostearate/Polyhydroxystearate/Sebacate | 3,000 | 3,000 | 3,000 | 3,000 | 3,000 |
| Parfum | 0,300 | 0,300 | 0,300 | 0,300 | 0,300 |

Der Ansatz setzt sich zusammen aus 75% Bulk und 25 % Treibgas (60% Butane + 20% Isobutane + 20% Propane, Druckstufe 2,7 bar)

| **Beispielrezeptur** | **46** | **47** | **48** | **49** | **50** |
|---|---|---|---|---|---|
| Ethylparaben | 0,100 | | | | |
| Methylparaben | 0,300 | | | | |
| Aqua | ad 100 | ad 100 | ad 100 | ad 100 | ad 100 |
| Magnesium Sulfate | 0,700 | 0,700 | 0,700 | 0,700 | 0,700 |
| Trisodium EDTA | 0,200 | 0,200 | 0,200 | 0,200 | 0,200 |
| Phenoxyethanol | 0,500 | 0,500 | 0,500 | 0,500 | 0,500 |
| Butyl Methoxydibenzoylmethane | 4,500 | 4,500 | 4,500 | 4,500 | 4,500 |
| Nylon-12 | 1,000 | | | | |
| Glycerin | 4,950 | 4,950 | 4,950 | 4,950 | 4,950 |
| Isopropyl Stearate | 12,000 | 12,000 | 12,000 | 12,000 | 6,000 |
| Alcohol Denat. | | 0,962 | 1,923 | | |
| Ethylhexyl Salicylate | 4,500 | 4,500 | 4,500 | 4,500 | 4,500 |
| Acrylates/Octylacrylamide Copolymer | | 1,000 | 1,000 | 1,000 | |
| Octocrylene | 9,500 | 9,500 | 9,500 | 9,500 | 9,500 |
| Homosalate | 9,500 | 9,500 | 9,500 | 9,500 | 9,500 |
| Bis-Ethylhexyloxyphenol Methoxyphenyl Triazine | 2,000 | 2,000 | 2,000 | 2,000 | 2,000 |
| Butylene Glycol Dicaprylate/Dicaprate | 8,500 | 8,500 | 8,500 | 8,500 | 8,500 |
| Potassium Sorbate | | 0,125 | 0,125 | 0,125 | 0,125 |
| C13-16 Isoparaffin | | | | | 6,000 |
| Polyglyceryl-4 Diisostearate/Polyhydroxystearate/Sebacate | 3,000 | 3,000 | 3,000 | 3,000 | 3,000 |
| Parfum | 0,300 | 0,300 | 0,300 | 0,300 | 0,300 |

Der Ansatz setzt sich zusammen aus 75% Bulk und 25% Treibgas (72% Isobutan +23% Propan + 5% n-Butan, Druckstufe 3,5 bar)

| **Beispielrezepturen** | **51** | **52** | **53** | **54** | **55** |
|---|---|---|---|---|---|
| Stearyl Alcohol | 0,5 | | | | |
| Butylene Glycol Dicaprylate/Dicaprate | 8,5 | 8,5 | 8,5 | 8,5 | 8,5 |
| Isopropyl Stearate | 12 | 12 | 12 | 12 | |
| Synthetic Beeswax | | | | 0,5 | |
| Triisostearin | | | | | 12 |
| Polyglyceryl-4 Diisostearate/Polyhydroxystearate/Sebacate | 3 | 3 | 3 | 3 | 3 |
| Acrylates/Octylacrylamide Copolymer | | 0,1 | | | |
| Parfum | 0,3 | 0,3 | 0,3 | 0,3 | 0,3 |
| Glycerin | 4,95 | 4,95 | 4,95 | 4,95 | 4,95 |
| Potassium Sorbate | 0,125 | 0,125 | 0,125 | 0,125 | 0,125 |
| Phenoxyethanol | 0,5 | 0,5 | 0,5 | 0,5 | 0,5 |
| Sodium Polyacrylate | | | 0,1 | | |
| Aqua | ad 100 | ad 100 | ad 100 | ad 100 | ad 100 |
| Alcohol Denat. | | 0,9617 | | | |
| Trisodium EDTA | 0,2 | 0,2 | 0,2 | 0,2 | 0,2 |
| Magnesium Sulfate | 0,7 | 0,7 | 0,7 | 0,7 | 0,7 |
| Homosalate | 9,5 | 9,5 | 9,5 | 9,5 | 9,5 |
| Octocrylene | 9,5 | 9,5 | 9,5 | 9,5 | 9,5 |
| Ethylhexyl Salicylate | 4,5 | 4,5 | 4,5 | 4,5 | 4,5 |
| Bis-Ethylhexyloxyphenol Methoxyphenyl Triazine | 2 | 2 | 2 | 2 | 2 |
| Butyl Methoxydibenzoylmethane | 4,5 | 4,5 | 4,5 | 4,5 | 4,5 |

Der Ansatz setzt sich zusammen aus 75% Bulk und 25 % Treibgas (60% Butane + 20% Isobutane + 20% Propane, Druckstufe 2,7 bar)

| **Beispielrezepturen** | **56** | **57** | **58** | **59** | **60** |
|---|---|---|---|---|---|
| Candelilla Cera | 0,25 | | | | |
| Butylene Glycol Dicaprylate/Dicaprate | 8,5 | 8,5 | 8,5 | 8,5 | 8,5 |
| Copernicia Cerifera Cera | 0,25 | | | | |
| Isopropyl Stearate | 12 | 12 | | | |
| C18-38 Alkyl Hydroxystearoyl Stearate | | 1 | | | |
| Squalane | | | 12 | | |
| Caprylic/Capric Triglyceride | | | | 12 | |
| Octyldodecanol | | | | | 12 |
| Polyglyceryl-4 Diisostearate/Polyhydroxystearate/Sebacate | 3 | 3 | 3 | 3 | 3 |
| Parfum | 0,3 | 0,3 | 0,3 | 0,3 | 0,3 |
| Glycerin | 4,95 | 4,95 | 4,95 | 4,95 | 4,95 |
| Potassium Sorbate | 0,125 | 0,125 | 0,125 | 0,125 | 0,125 |
| Phenoxyethanol | 0,5 | 0,5 | 0,5 | 0,5 | 0,5 |
| Aqua | ad 100 | ad 100 | ad 100 | ad 100 | ad 100 |
| Trisodium EDTA | 0,2 | 0,2 | 0,2 | 0,2 | 0,2 |
| Magnesium Sulfate | 0,7 | 0,7 | 0,7 | 0,7 | 0,7 |
| Homosalate | 9,5 | 9,5 | 9,5 | 9,5 | 9,5 |
| Octocrylene | 9,5 | 9,5 | 9,5 | 9,5 | 9,5 |
| Ethylhexyl Salicylate | 4,5 | 4,5 | 4,5 | 4,5 | 4,5 |
| Bis-Ethylhexyloxyphenol Methoxyphenyl Triazine | 2 | 2 | 2 | 2 | 2 |
| Butyl Methoxydibenzoylmethane | 4,5 | 4,5 | 4,5 | 4,5 | 4,5 |

Der Ansatz setzt sich zusammen aus 75% Bulk und 25% Treibgas (72% Isobutan +23% Propan + 5% n-Butan, Druckstufe 3,5 bar)

| **Beispielrezepturen** | **61** | **62** | **63** | **64** | **65** |
|---|---|---|---|---|---|
| Isohexadecane | 8,5 | | | | |
| Isopropyl Stearate | 12 | 12 | 12 | 6 | 12 |
| C12-15 Alkyl Benzoate | | 8,5 | | | |
| Butylene Glycol Dicaprylate/Dicaprate | | | 8,5 | 8,5 | 8,5 |
| Ethylhexyl Cocoate | | | | 6 | |
| Polyglyceryl-4 Diisostearate/Polyhydroxystearate/Sebacate | 3 | 3 | 3 | 3 | 3 |
| Silica Dimethyl Silylate | | | 0,5 | | |
| Parfum | 0,3 | 0,3 | 0,3 | 0,3 | 0,3 |
| Glycerin | 4,95 | 4,95 | 4,95 | 4,95 | 4,95 |
| Potassium Sorbate | 0,125 | 0,125 | 0,125 | 0,125 | 0,125 |
| Phenoxyethanol | 0,5 | 0,5 | 0,5 | 0,5 | 0,5 |
| Aqua | ad 100 | ad 100 | ad 100 | ad 100 | ad 100 |
| Trisodium EDTA | 0,2 | 0,2 | 0,2 | 0,2 | 0,2 |
| Magnesium Sulfate | 0,7 | 0,7 | 0,7 | 0,7 | 0,7 |
| Homosalate | 9,5 | 9,5 | 9,5 | 9,5 | 9,5 |
| Octocrylene | 9,5 | 9,5 | 9,5 | 9,5 | 9,5 |
| Ethylhexyl Salicylate | 4,5 | 4,5 | 4,5 | 4,5 | 4,5 |
| Bis-Ethylhexyloxyphenol Methoxyphenyl Triazine | 2 | 2 | 2 | 2 | 2 |
| Butyl Methoxydibenzoylmethane | 4,5 | 4,5 | 4,5 | 4,5 | 4,5 |
| Titanium Dioxide (nano) + Silica | | | | | 1 |

Der Ansatz setzt sich zusammen aus 75% Bulk und 25 % Treibgas (60% Butane + 20% Isobutane + 20% Propane, Druckstufe 2,7 bar)

## Patentansprüche

1. Kosmetisches Aerosolspray enthaltend eine W/O-Emulsion enthaltend
a) Polyglyceryl-4 Diisostearate/Polyhydroxystearate/Sebacate sowie
b) einen oder mehrere UV-Filter,
**dadurch gekennzeichnet, dass** die W/O-Emulsion mit einem Treibgas versetzt ist, wobei das Treibgas gewählt wird aus Propan, n-Butan, Isobutan und deren Mischungen.

2. Kosmetisches Aerosolspray nach Anspruch 1 **dadurch gekennzeichnet, dass** die W/O-Emulsion Diisostearoyl Polyglyceryl-3 Dimer Dioleate enthält.

3. Kosmetisches Aerosolspray nach einem der vorhergehenden Ansprüche, **dadurch gekennzeichnet, dass** die W/O-Emulsion einen oder mehrere UV-Filter enthält, gewählt aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 4-(tert.-Butyl)-4'-methoxydibenzoylmethan; 2-Ethylhexyl-2-cyano-3,3-diphenylacrylat; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone); 2,4-bis-[5-1 (dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4;6-triyltrümino)-tris-benzoesäure-tris-(2-ethylhexylester) (auch: 2,4,6-Tris-[anilino-(p-carbo-2'-ethyl-1'-hexyloxy)]-1,3,5-triazin (INCI: Ethylhexyl Triazone); 2,4-Bis-{[4-(2-ethyl-hexyloxy)-2-hydroxy]-phenyl}-6-(4-methoxyphenyl)-1,3,5-triazin; 2,4,6-Tribiphenyl-4-yl-1,3,5-triazin; Merocyanine; Titandioxid; Zinkoxid.

4. Kosmetisches Aerosolspray nach einem der vorhergehenden Ansprüche, **dadurch gekennzeichnet, dass** die W/O-Emulsion 4-(tert.-Butyl)-4'-methoxydibenzoylmethan und/oder 2-(4'-Diethylamino-2'-hydoxybenzoyl)-benzoesäurehexylester enthält.

5. Kosmetisches Aerosolspray nach einem der vorhergehenden Ansprüche, **dadurch gekennzeichnet, dass** die W/O-Emulsion einen oder mehrere UV-Filter gewählt aus der Gruppe der Verbindungen der Triazine enthält.

6. Kosmetisches Aerosolspray nach einem der vorhergehenden Ansprüche, **dadurch gekennzeichnet, dass** die W/O-Emulsion 2,4-Bis-{[4-(2-ethyl-hexyloxy)-2-hydroxy]-phenyl}-6-(4-methoxyphenyl)-1,3,5-triazin enthält.

7. Kosmetisches Aerosolspray nach einem der vorhergehenden Ansprüche, **dadurch gekennzeichnet, dass** die W/O-Emulsion Octocrylen enthält.

8. Kosmetisches Aerosolspray nach einem der vorhergehenden Ansprüchen, **dadurch gekennzeichnet, dass** die W/O-Emulsion Polyglyceryl-4 Diisostearate/Polyhydroxystearate/Sebacate in einer Konzentration von 0,5 bis 5 Gewichts-%, bezogen auf das Gesamtgewicht der W/O=Emulsion, enthält.

9. Kosmetisches Aerosolspray nach einem der vorhergehenden Ansprüche, **dadurch gekennzeichnet, dass** die W/O-Emulsion Diisostearoyl Polyglyceryl-3 Dimer Dioleate in einer Konzentration von 0,5 bis 5 Gewichts-% bezogen auf das Gesamtgewicht der W/O-Emulsion, enthält.

10. Kosmetisches Aerosolspray nach einem der vorhergehenden Ansprüche, **dadurch gekennzeichnet, dass** die W/O-Emulsion frei ist von Polyethylenglycolen.

11. Kosmetisches Aerosolspray nach einem der vorhergehenden Ansprüche, **dadurch gekennzeichnet, dass** die W/O-Emulsion einen oder mehrere Wirkstoffe gewählt aus der Gruppe der Verbindungen Gylcyrrhetinsäure, Harnstoff, Arctiin, alpha-Liponsäure, Folsäure, Phytoen, D-Biotin, Coenzym Q10, alpha-Glucosylrutin, Carnitin, Carnosin, Coffein, natürliche und/oder synthetische Isoflavonoide, Glycerylglucose, Kreatin, Kreatinin, Taurin, Tocopherol, Tocopherolacetat, ß-Älanin und/oder Licochalcon A, enthält.

12. Kosmetisches Aerosolspray nach einem der vorhergehenden Ansprüche, **dadurch gekennzeichnet, dass** die W/O-Emulsion Propylenglycol, Butylenglycol, 2-Methylpropan-1,3-diol, 1,2-Pentandiol, 1,2-Hexandiol, 1,2-Octandiol und/oder 1,2-Decandiol enthält.

13. Kosmetisches Aerosolspray nach einem der vorhergehenden Ansprüche, **dadurch gekennzeichnet, dass** die W/O-Emulsion Phenoxyethanol enthält.

14. Kosmetisches Aerosolspray nach einem der vorhergehenden Ansprüche, **dadurch gekennzeichnet, dass** die W/O-Emulsion Xanthangummi enthält.

15. Kosmetisches Aerosolspray nach einem der vorhergehenden Ansprüche, **dadurch gekennzeichnet, dass** die W/O-Emulsion Füllstoffe, insbesondere Polyethylen, Nylon, natürliche oder modifizierte Stärken wie Tapiokastärke und/oder Silikate wie beispielsweise Talkum enthält.

16. Kosmetisches Aerosolspray nach einem der vorhergehenden Ansprüche, **dadurch gekennzeichnet, dass** die W/O-Emulsion frei ist von Parabenen.

17. Kosmetisches Aerosolspray nach einem der vorhergehenden Ansprüchen, **dadurch gekennzeichnet, dass** die W/O-Emulsion frei ist von 3-(4-Methylbenzyliden)campher.

18. Treibgasdose enthaltend ein Aerosolspray nach einem der vorhergehenden Ansprüchen, **dadurch gekennzeichnet, dass** die Dose eine Aluminiumdose ist, die auf der Innenseite mit einem Schutzlack beschichtet ist.

19. Treibgasdose nach Anspruch 18, **dadurch gekennzeichnet, dass** der Schutzlack ein Polyamid-Imid-Lack oder ein Pulverlack ist.

20. Treibgasdose nach Anspruch 18 oder 19, **dadurch gekennzeichnet, dass** das Ventil im Sprühkopf ein Ariane Kugelventil ist.

## Claims

1. Cosmetic aerosol spray containing a W/O emulsion containing
a) polyglyceryl-4 diisostearate/ polyhydroxystearate/sebacate and
b) one or more UV filters,
**characterized in that** the W/O emulsion is admixed with a propellant, the propellant being selected from propane, n-butane, isobutane and the mixtures thereof.

2. Cosmetic aerosol spray according to Claim 1, **characterized in that** the W/O emulsion contains diisostearoyl polyglyceryl-3 dimer dioleate.

3. Cosmetic aerosol spray according to either of the preceding claims, **characterized in that** the W/O emulsion contains one or more UV filters selected from the group of the compounds 2-phenylbenzimidazole-5-sulfonic acid and/or the salts thereof; phenylene-1,4-bis(2-benzimidazyl)-3,3'-5,5'-tetrasulfonic acid salts; 1,4-di(2-oxo-10-sulfo-3-bornylidenemethyl)benzene and the salts thereof; 4-(2-oxo-3-bornylidenemethyl)benzenesulfonic acid salts; 2-methyl-5-(2-oxo-3-bornylidenemethyl)sulfonic acid salts; 2,2'-methylenebis(6-(2H-benzotriazol-2-yl)-4-(1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]disiloxanyl]propyl]phenol; 3-(4-methylbenzylidene)camphor; 3-benzylidenecamphor; ethylhexyl salicylate; terephthalidenedicamphorsulfonic acid; 4-(tert-butyl)-4'-methoxydibenzoylmethane; 2-ethylhexyl 2-cyano-3,3-diphenylacrylate; 2-ethylhexyl 4-(dimethylamino)benzoate; amyl 4-(dimethylamino)benzoate; di(2-ethylhexyl) 4-methoxybenzalmalonate; 2-ethylhexyl 4-methoxycinnamate; isoamyl 4-methoxycinnamate; 2-hydroxy-4-methoxybenzophenone, 2-hydroxy-4-methoxy-4'-methylbenzophenone; 2,2'-dihydroxy-4-methoxybenzophenone; hexyl 2-(4'-diethylamino-2'-hydroxybenzoyl)benzoate; homomenthyl salicylate; 2-ethylhexyl 2-hydroxybenzoate; dimethicodiethyl benzalmalonate; 3-(4-(2,2-bisethoxycarbonylvinyl)phenoxy)propenyl)methoxy-siloxane/dimethylsiloxane copolymer; dioctylbutylamidotriazone (INCI: diethylhexyl butamido triazone); 2,4-bis[5-1 (dimethylpropyl)benzoxazol-2-yl-(4-phenyl)-imino]-6-(2-ethylhexyl)imino-1,3,5-triazine with the (CAS No. 288254-16-0); tris(2-ethylhexyl) 4,4',4"-(1,3,5-triazine-2,4,6-triyltriimino)trisbenzoate (also: 2,4,6-tris[anilino(p-carbo-2'-ethyl-1'-hexyloxy)]-1,3,5-triazine (INCI: ethylhexyl triazone); 2,4-bis{[4-(2-ethylhexyloxy)-2-hydroxy]phenyl}-6-(4-methoxyphenyl)-1,3,5-triazine; 2,4,6-tribiphenyl-4-yl-1,3,5-triazine; merocyanines; titanium dioxide; zinc oxide.

4. Cosmetic aerosol spray according to any of the preceding claims, **characterized in that** the W/O emulsion contains 4-(tert-butyl)-4'-methoxydibenzoylmethane and/or hexyl 2-(4'-diethylamino-2'-hydroxybenzoyl)benzoate.

5. Cosmetic aerosol spray according to any of the preceding claims, **characterized in that** the W/O emulsion contains one or more UV filters selected from the group of the compounds of the triazines.

6. Cosmetic aerosol spray according to any of the preceding claims, **characterized in that** the W/O emulsion contains 2,4-bis{[4-(2-ethylhexyloxy)-2-hydroxy]phenyl}-6-(4-methoxyphenyl)-1,3,5-triazine.

7. Cosmetic aerosol spray according to any of the preceding claims, **characterized in that** the W/O emulsion contains octocrylene.

8. Cosmetic aerosol spray according to any of the preceding claims, **characterized in that** the W/O emulsion contains polyglyceryl-4 diisostearate/
polyhydroxystearate/sebacate in a concentration of from 0.5 to 5% by weight, based on the total weight of the W/O emulsion.

9. Cosmetic aerosol spray according to any of the preceding claims, **characterized in that** the W/O emulsion contains diisostearoyl polyglyceryl-3 dimer dioleate in a concentration of from 0.5 to 5% by weight, based on the total weight of the W/O emulsion.

10. Cosmetic aerosol spray according to any of the preceding claims, **characterized in that** the W/O emulsion is free of polyethylene glycols.

11. Cosmetic aerosol spray according to any of the preceding claims, **characterized in that** the W/O emulsion contains one or more active ingredients selected from the group of the compounds glycyrrhetinic acid, urea, arctiin, alpha-lipoic acid, folic acid, phytoene, D-biotin, coenzyme Q10, alpha-glucosylrutin, carnitine, carnosine, caffeine, natural and/or synthetic isoflavonoids, glyceryl glucose, creatine, creatinine, taurine, tocopherol, tocopherol acetate, β-alanine and/or licochalcone A.

12. Cosmetic aerosol spray according to any of the preceding claims, **characterized in that** the W/O emulsion contains propylene glycol, butylene glycol, 2-methylpropane-1,3-diol, 1,2-pentanediol, 1,2-hexanediol, 1,2-octanediol and/or 1,2-decanediol.

13. Cosmetic aerosol spray according to any of the preceding claims, **characterized in that** the W/O emulsion contains phenoxyethanol.

14. Cosmetic aerosol spray according to any of the preceding claims, **characterized in that** the W/O emulsion contains xanthan gum.

15. Cosmetic aerosol spray according to any of the preceding claims, **characterized in that** the W/O emulsion contains fillers, more particularly polyethylene, nylon, natural or modified starches such as tapioca starch and/or silicates such as, for example, talc.

16. Cosmetic aerosol spray according to any of the preceding claims, **characterized in that** the W/O emulsion is free of parabens.

17. Cosmetic aerosol spray according to any of the preceding claims, **characterized in that** the W/O emulsion is free of 3-(4-methylbenzylidene)camphor.

18. Propellant can containing an aerosol spray according to any of the preceding claims, **characterized in that** the can is an aluminium can coated on the inner side with a protective coating.

19. Propellant can according to Claim 18, **characterized in that** the protective coating is a polyamide-imide coating or a powder coating.

20. Propellant can according to Claim 18 or 19, **characterized in that** the valve in the spray head is an Ariane ball valve.

## Revendications

1. Spray aérosol cosmétique contenant une émulsion E/H contenant :
a) du Polyglyceryl-4 Diisostearate/Polyhydroxystearate/Sebacate et
b) un ou plusieurs filtres UV,
**caractérisé en ce que** l'émulsion E/H est mélangée avec un gaz propulseur, le gaz propulseur étant choisi parmi le propane, le n-butane, l'isobutane et leurs mélanges.

2. Spray aérosol cosmétique selon la revendication 1, **caractérisé en ce que** l'émulsion E/H contient du Diisostearoyl Polyglyceryl-3 Dimer Dioleate.

3. Spray aérosol cosmétique selon l'une quelconque des revendications précédentes, **caractérisé en ce que** l'émulsion E/H contient un ou plusieurs filtres UV choisis dans le groupe des composés acide 2-phénylbenzimidazole-5-sulfonique et/ou ses sels ; sels de l'acide phénylène-1,4-bis-(2-benzimidazyl)-3,3'-5,5'-tétrasulfonique ; 1,4-di(2-oxo-10-sulfo-3-bornylidène-méthyl)-benzène et ses sels ; sels de l'acide 4-(2-oxo-3-bornylidène-méthyl)benzène-sulfonique ; sels de l'acide 2-méthyl-5-(2-oxo-3-bornylidène-méthyl)sulfonique ; 2,2'-méthylène-bis-(6-(2H-benzotriazol-2-yl)-4-(1,1,3,3-tétraméthylbutyl)-phénol) ; 2-(2H-benzotriazol-2-yl)-4-méthyl-6-[2-méthyl-3-[1,3,3,3-tétraméthyl-1-[(triméthylsilyl)oxy]disiloxanyl]propyl]-phénol ; 3-(4-méthylbenzylidène)camphre ; 3-benzylidène-camphre ; salicylate d'éthylhexyle ; acide téréphtalidène-dicamphre-sulfonique ; 4-(tert.-butyl)-4'-méthoxydibenzoylméthane ; acrylate de 2-éthylhexyl-2-cyano-3,3-diphényle ; ester 2-éthylhexylique de l'acide 4-(diméthylamino)-benzoïque ; ester amylique de l'acide 4-(diméthylamino)benzoïque ; ester di(2-éthylhexylique) de l'acide 4-méthoxybenzalmalonique ; ester 2-éthylhexylique de l'acide 4-méthoxycinnamique ; ester isoamylique de l'acide 4-méthoxycinnamique ; 2-hydroxy-4-méthoxybenzophénone, 2-hydroxy-4-méthoxy-4'-méthylbenzophénone ; 2,2'-dihydroxy-4-méthoxybenzophénone ; ester hexylique de l'acide 2-(4'-diéthylamino-2'-hydroxybenzoyl)-benzoïque ; salicylate d'homomenthyle ; 2-hydroxybenzoate de 2-éthylhexyle ; benzalmalonate de diméthicodiéthyle ; copolymère de 3-(4-(2,2-bis-éthoxycarbonylvinyl)-phénoxy)propényl)-méthoxysiloxane/diméthylsiloxane ; dioctylbutylamidotriazone (INCI : Diethylhexyl-Butamidotriazone) ; 2,4-bis-[5-1(diméthyl-propyl)benzoxazol-2-yl-(4-phényl)-imino]-6-(2-éthylhexyl)-imino-1,3,5-triazine de n° CAS 288254-16-0 ; ester tris-(2-éthylhexylique) de l'acide 4,4',4"-(1,3,5-triazine-2,4,6-triyltriimino)-tris-benzoïque (également : 2,4,6-tris-[anilino-(p-carbo-2'-éthyl-1'-hexyloxy)]-1,3,5-triazine (INCI : Ethylhexyl Triazone) ; 2,4-bis-{[4-(2-éthyl-hexyloxy)-2-hydroxy]-phényl}-6-(4-méthoxyphényl)-1,3,5-triazine ; 2,4,6-tribiphényl-4-yl-1,3,5-triazine ; mérocyanine ; dioxyde de titane ; oxyde de zinc.

4. Spray aérosol cosmétique selon l'une quelconque des revendications précédentes, **caractérisé en ce que** l'émulsion E/H contient du 4-(tert.-butyl)-4'-méthoxydibenzoylméthane et/ou de l'ester hexylique de l'acide 2-(4'-diéthylamino-2'-hydroxybenzoyl)-benzoïque.

5. Spray aérosol cosmétique selon l'une quelconque des revendications précédentes, **caractérisé en ce que** l'émulsion E/H contient un ou plusieurs filtres UV choisis dans le groupe des composés de triazines.

6. Spray aérosol cosmétique selon l'une quelconque des revendications précédentes, **caractérisé en ce que** l'émulsion E/H contient de la 2,4-bis-{[4-(2-éthyl-hexyloxy)-2-hydroxy]-phényl}-6-(4-méthoxyphényl)-1,3,5-triazine.

7. Spray aérosol cosmétique selon l'une quelconque des revendications précédentes, **caractérisé en ce que** l'émulsion E/H contient de l'octocrylène.

8. Spray aérosol cosmétique selon l'une quelconque des revendications précédentes, **caractérisé en ce que** l'émulsion E/H contient du Polyglyceryl-4 Diisostearate/Polyhydroxystearate/Sebacate en une concentration de 0,5 à 5 % en poids, par rapport au poids total de l'émulsion E/H.

9. Spray aérosol cosmétique selon l'une quelconque des revendications précédentes, **caractérisé en ce que** l'émulsion E/H contient du Diisostearoyl Polyglyceryl-3 Dimer Dioleate en une concentration de 0,5 à 5 % en poids, par rapport au poids total de l'émulsion E/H.

10. Spray aérosol cosmétique selon l'une quelconque des revendications précédentes, **caractérisé en ce que** l'émulsion E/H est exempte de polyéthylène glycols.

11. Spray aérosol cosmétique selon l'une quelconque des revendications précédentes, **caractérisé en ce que** l'émulsion E/H contient un ou plusieurs agents actifs choisis dans le groupe des composés acide glycyrrhétique, urée, arctiine, acide alpha-liponique, acide folique, phytoène, D-biotine, coenzyme Q10, alpha-glucosylrutine, carnitine, carnosine, caféine, isoflavonoïdes naturels et/ou synthétiques, glycérylglucose, créatine, créatinine, taurine, tocophérol, acétate de tocophérol, β-alanine et/ou licochalcone A.

12. Spray aérosol cosmétique selon l'une quelconque des revendications précédentes, **caractérisé en ce que** l'émulsion E/H contient du propylène glycol, du butylène glycol, du 2-méthylpropane-1,3-diol, du 1,2-pentanediol, du 1,2-hexanediol, du 1,2-octanediol et/ou du 1,2-décanediol.

13. Spray aérosol cosmétique selon l'une quelconque des revendications précédentes, **caractérisé en ce que** l'émulsion E/H contient du phénoxyéthanol.

14. Spray aérosol cosmétique selon l'une quelconque des revendications précédentes, **caractérisé en ce que** l'émulsion E/H contient de la gomme xanthane.

15. Spray aérosol cosmétique selon l'une quelconque des revendications précédentes, **caractérisé en ce que** l'émulsion E/H contient des charges, notamment du polyéthylène, du nylon, des amidons naturels ou modifiés tels que l'amidon de tapioca et/ou des silicates tels que par exemple le talc.

16. Spray aérosol cosmétique selon l'une quelconque des revendications précédentes, **caractérisé en ce que** l'émulsion E/H est exempte de parabènes.

17. Spray aérosol cosmétique selon l'une quelconque des revendications précédentes, **caractérisé en ce que** l'émulsion E/H est exempte de 3-(4-méthylbenzylidène)camphre.

18. Boîte de gaz propulseur contenant un spray aérosol selon l'une quelconque des revendications précédentes, **caractérisée en ce que** la boîte est une boîte en aluminium qui est revêtue sur le côté intérieur avec un vernis protecteur.

19. Boîte de gaz propulseur selon la revendication 18, **caractérisée en ce que** le vernis protecteur est un vernis polyamide-imide ou un vernis en poudre.

20. Boîte de gaz propulseur selon la revendication 18 ou 19, **caractérisée en ce que** le clapet dans la tête de pulvérisation est un clapet à bille Ariane.
